# EUROPÄISCHE PATENTSCHRIFT

(11) **EP 0 906 267 B1**
(45) Veröffentlichungstag und Bekanntmachung des Hinweises auf die Patenterteilung: **31.10.2001**
(21) Anmeldenummer: 97928163.1
(22) Anmeldetag: 09.06.1997
(51) Int. Cl.: C07C 209/88

(54) **VERFAHREN ZUR HERSTELLUNG VON OPTISCH AKTIVEN 1-PHENYL-ETHYLAMINEN**
METHOD FOR PRODUCING OPTICALLY ACTIVE 1-PHENYLETHYLAMINES
PROCEDE POUR PRODUIRE DES 1-PHENYLETHYLAMINES OPTIQUEMENT ACTIVES

(30) Priorität: 21.06.1996 DE 19624820
(43) Veröffentlichungstag der Anmeldung: 07.04.1999
(73) Patentinhaber: BAYER AG, 51368 Leverkusen (DE)
(72) Erfinder: MERZ, Walter, D-51375 Leverkusen (DE); LITTMANN, Martin, D-51375 Leverkusen (DE); KRAATZ, Udo, D-51375 Leverkusen (DE); MANNHEIMS, Christoph, D-51373 Leverkusen (DE)
(86) Internationale Anmeldenummer: EP9702988
(87) Internationale Veröffentlichungsnummer: WO9749665

(56) Entgegenhaltungen:
- EP-A- 0 341 475
- DE-A- 4 039 447
- E. BROWN ET AL: "A new chiral acid for the resolution of racemic bases : (S)-(-)-(2-phenylcarbamoyloxy)propionic acid (carbamalactic acid)" TETRAHEDRON LETTERS, Bd. 26, Nr. 37, 1985, Seiten 4451-4452, XP002042446

## Beschreibung

Die vorliegende Erfindung betrifft ein neues Verfahren zur Herstellung von bekannten, optisch aktiven 1-Phenylethylaminen, die als Zwischenprodukte zur Synthese von Wirkstoffen mit fungiziden oder pharmakologischen Eigenschaften verwendbar sind.

Es ist bereits bekannt geworden, daß sich (R)-1-(4-Chlor-phenyl)-ethylamin herstellen läßt, indem man racemisches 1-(4-Chlor-phenyl)-ethylamin in Gegenwart von Ethanol mit S-(-)-N-Phenylcarbamat-milchsäure umsetzt, den dabei entstehenden Kristallbrei absaugt und mit wäßriger Natronlauge in Gegenwart von Methylenchlorid behandelt (vgl. EP-A 0 341 475). Nachteilig an diesem Verfahren ist, daß ein beachtlicher Anteil an S-(-)-Phenylcarbamat-milchsäure in der nach dem Absaugen des Kristallbreis anfallenden Mutterlauge vorhanden ist und daraus nur in aufwendiger Weise isoliert werden kann.

Weiterhin ist schon bekannt, daß sich (S)-1-(4-Chlor-phenyl)-ethylamin gewinnen läßt, indem man die bei dem zuvor erwähnten Verfahren anfallende Mutterlauge durch Abdestillieren von Ethanol einengt, den verbleibenden Rückstand mit tert.-Butyl-methylether oder Toluol verrührt, das sich abscheidende kristalline Salz abtrennt und die Mutterlauge destilliert (vgl. DE-A 4 039 447). Dieses Verfahren ist aber für die Durchführung im technischen Maßstab zu aufwendig.

Es wurde nun gefunden, daß man optisch aktive 1-Phenyl-ethyl-amine der Formel 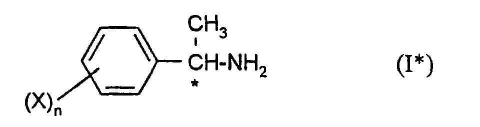 in welcher
- X: für Halogen oder Methyl steht, und
- n: für die Zahlen 0, 1 oder 2 steht,
aus racemischen 1-Phenyl-ethyl-aminen mit Hilfe von (S)-(-)-N-Phenylcarbamatmilchsäure in separater Form erhält, wenn man
a) racemische 1-Phenyl-ethylamine der Formel 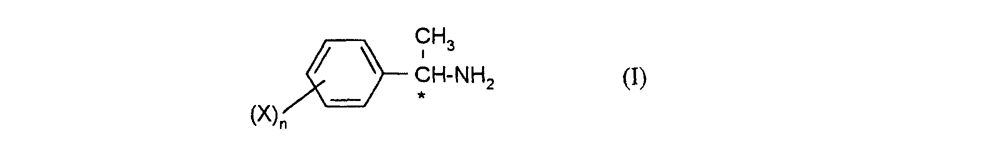 in welcher
   - X und n: die oben angegebenen Bedeutungen haben,
   mit (S)-(-)-N-Phenylcarbamat-milchsäure der Formel 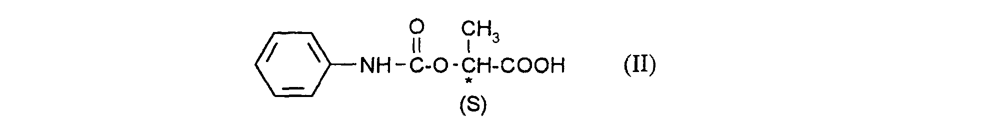 in Gegenwart eines aliphatischen oder aromatischen Kohlenwasserstoffes und in Gegenwart eines niederen aliphatischen Alkohols umsetzt, wobei die Mengen an den Reaktionskomponenten so bemessen sind, daß auf 1 Mol an racemischem 1-Phenyl-ethyl-amin der Formel (I) zwischen 0,25 und 0,5 Mol an (S)-(-)-N-Phenylcarbamat-milchsäure der Formel (II) vorhanden sind,
   danach den niederen aliphatischen Alkohol sowie gegebenenfalls einen Teil des aliphatischen oder aromatischen Kohlenwasserstoffes bei einer Sumpftemperatur bis zu 40°C gegebenenfalls unter vermindertem Druck abdestilliert,
   das dabei entstehende kristalline Produkt der Formel 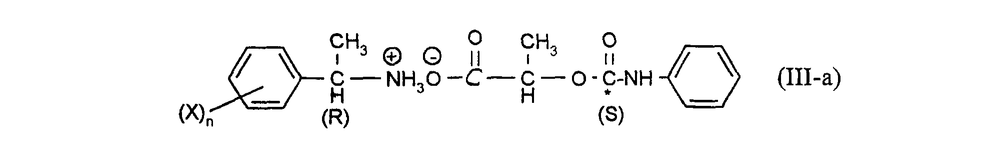 in welcher
   - X und n: die oben angegebenen Bedeutungen haben,
   abtrennt, mit verdünnter, wäßriger Alkalilauge in Gegenwart eines aliphatischen oder aromatischen Kohlenwasserstoffes verrührt, die organische Phase abtrennt, die wäßrige Phase erneut mit einem aliphatischen oder aromatischen Kohlenwasserstoff verrührt, die organische Phase wiederum abtrennt und aus den vereinigten organischen Phasen das jeweils entstandene (R)-1-Phenyl-ethylamin der Formel 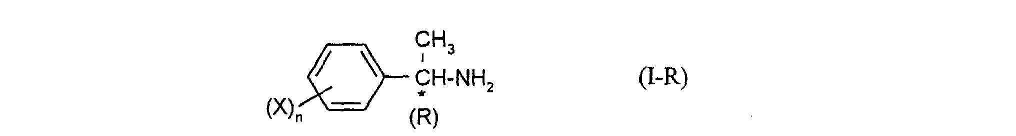 in welcher
   - X und n: die oben angegebenen Bedeutungen haben,
   destillativ isoliert,
   und gegebenenfalls
b) die nach der Abtrennung des Salzes der Formel (III-a) verbliebene Mutterlauge in Gegenwart eines niederen aliphatischen Alkohols mit (S)-(-)-N-Phenylcarbamat-milchsäure der Formel 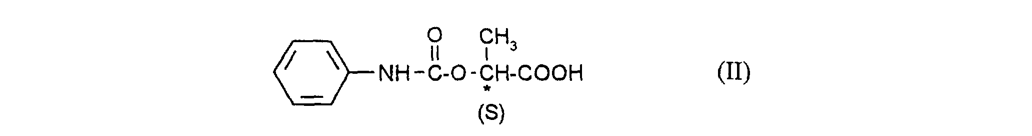 umsetzt, wobei die Mengen an den Reaktionskomponenten so bemessen sind, daß die molare Menge an (S)-(-)-N-Phenylcarbamat-milchsäure der Formel (II) doppelt so groß ist wie die in der Mutterlauge noch vorhandene Menge an (R)-1-Phenyl-ethylamin der Formel (I-R), danach den niederen aliphatischen Alkohol sowie gegebenenfalls einen Teil des aliphatischen oder aromatischen Kohlenwasserstoffes bei einer Sumpftemperatur bis zu 40°C gegebenenfalls unter vermindertem Druck abdestilliert,
   das dabei entstehende kristalline Produkt der Formel 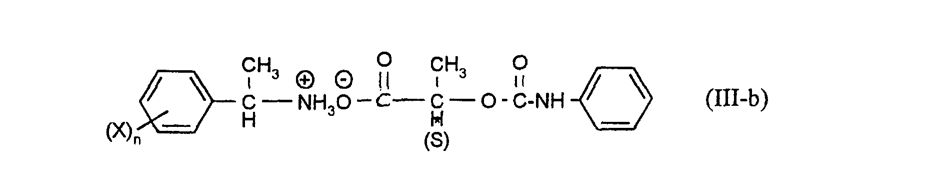 in welcher
   - X und n: die oben angegebenen Bedeutungen haben,
   abtrennt und aus der verbleibenden Mutterlauge das jeweils entstandene (S)-1-Phenyl-ethylamin der Formel 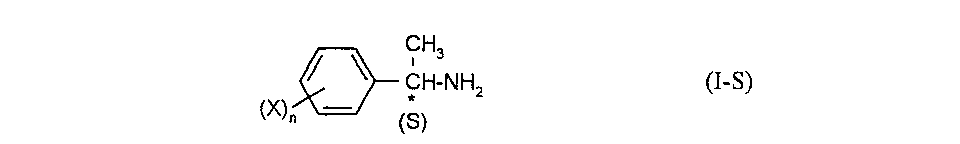 in welcher
   - X und n: die oben angegebenen Bedeutungen haben,
   destillativ isoliert.

Zur Kennzeichnung optisch aktiver Verbindungen sind die Chiralitätszentren in den obigen Formeln und auch weiterhin jeweils durch (*) markiert. Die Konfiguration der asymmetrisch substituierten Kohlenstoffatome wird durch die Symbole (R) oder (S) angegeben.

Es ist als äußerst überraschend zu bezeichnen, daß sich (R)-1-Phenyl-ethylamine der Formel (I-R) nach dem erfindungsgemäßen Verfahren in extrem hoher Ausbeute und hervorragender optischer Reinheit herstellen lassen. Aufgrund des bekannten Standes der Technik war nämlich nicht damit zu rechnen, daß aus dem nach dem Abdestillieren des niederen aliphatischen Alkohols vorhandenen Kohlenwasserstoff bevorzugt das jeweilige Salz aus (R)-1-Phenyl-ethylamin und (S)-(-)-N-Phenylcarbamat-milchsäure ausfällt, denn sowohl die Salze aus (R)-1-Phenyl-ethylaminen und (S)-(-)-N-Phenylcarbamat-milchsäure als auch die Salze aus (S)-1-Phenyl-ethylaminen und (S)-(-)-N-Phenylcarbamat-milchsäure sind in dem Kohlenwasserstoff gleichermaßen wenig löslich. Unerwartet ist außerdem, daß man aus der nach dem Abtrennen des Salzes der Formel (III-a) erhaltenen Mutterlauge durch erneute Zugabe von (S)-(-)-Phenylcarbamat-milchsäure das entsprechende Salz des racemischen 1-Phenyl-ethylamins praktisch quantitativ ausfällen kann und aus dem Filtrat das jeweilige (S)-1-Phenyl-ethylamin in hoher optischer Reinheit isolieren kann. Überraschend ist vor allem, daß sich das Kristallisationsverhalten der isomeren Salze beim erfindungsgemäßen Arbeiten in zwei Stufen völlig unterscheidet von demjenigen, das bei der analogen einstufigen Reaktion beobachtet wird. Setzt man nämlich racemisches 1-(4-Chlorphenyl)-ethylamin mit beliebigen Mengen an (S)-(-)-N-Phenylcarbamat-milchsäure in einer einzigen Stufe um, so gelingt es nach der Abtrennung des Salzes in keinem Fall, das in der Mutterlauge vorhandene (S)-1-(4-Chlor-phenyl)-ethylamin in hoher optischer Reinheit zu isolieren. Im übrigen war nicht vorherzusehen, daß das erfindungsgemäße Verfahren nur dann in der gewünschten Weise abläuft, wenn die Reaktionskomponenten in den speziell angegebenen molaren Mengen eingesetzt werden.

Das erfindungsgemäße Verfahren zeichnet sich durch eine Reihe von Vorteilen aus. So ermöglicht es, wie bereits erwähnt, die Herstellung von (R)-1-Phenylethylaminen der Formel (I-R) in extrem hoher Ausbeute und hervorragender Reinheit. Von besonderem Vorteil ist dabei, daß die eingesetzte (S)-(-)-N-Phenylcarbamat-milchsäure in einfacher Weise praktisch quantitativ zurückgewonnen und erneut verwendet werden kann. Außerdem ist die erfindungsgemäße Racematspaltung wesentlich weniger aufwendig als die entsprechenden vorbekannten Methoden. Günstig ist auch, daß die nach dem Abtrennen des Salzes im zweiten Reaktionsschritt anfallende Mutterlauge das jeweilige (S)-1-Phenyl-ethylamin nahezu vollständig enthält und eine problemlose Isolierung dieser Substanzen gestattet. Schließlich kann aus dem in der zweiten Stufe abgetrennten Salz der enthaltene Anteil an racemischem 1-Phenyl-ethylamin weitgehend und mühelos zurückgewonnen werden.

Setzt man 1 Mol racemisches 1-(4-Chlorphenyl)-ethylamin in der ersten Stufe mit 0,45 Mol an (S)-(-)-N-Phenylcarbamat-milchsäure in Gegenwart von Toluol und Methanol um, verwendet man wäßrige Natronlauge zur Freisetzung des (R)-1-(4-Chlor-phenyl)-ethylamins und setzt man in der zweiten Stufe mit 0,2 Mol (S)-(-)-N-Phenylcarbamat-milchsäure in Gegenwart von Toluol und Methanol um, so kann der Verlauf des erfindungsgemäßen Verfahrens durch das folgende Formelschema veranschaulicht werden:

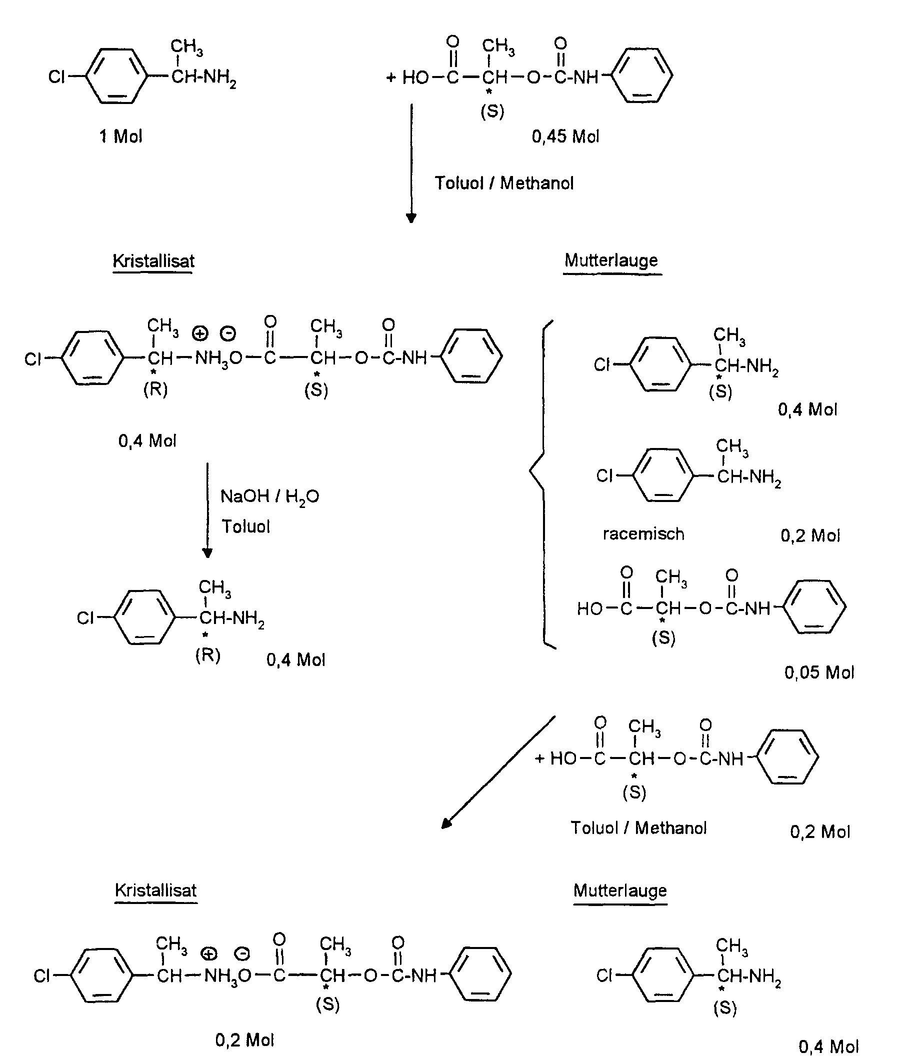

Die bei der Durchführung des erfindungsgemäßen Verfahrens als Ausgangssubstanzen benötigten racemischen 1-Phenyl-ethylamine sind durch die Formel (I) definiert. In dieser Formel steht X vorzugsweise für Fluor, Chlor, Brom oder Methyl. Der Index n steht für die Zahlen 0, 1 oder 2.

Als Beispiele für racemische 1-Phenyl-ethylamine der Formel (I) seien die folgenden Verbindungen genannt:

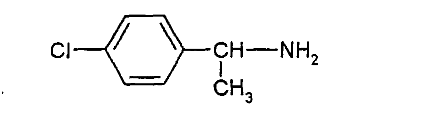

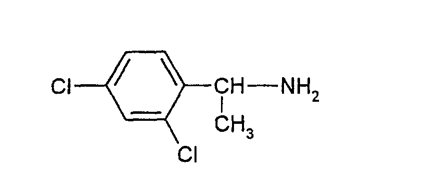

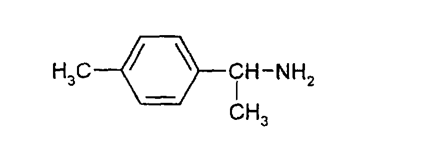

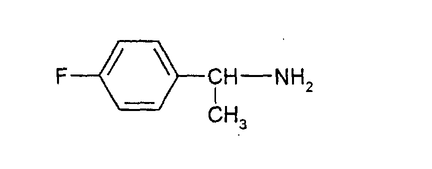

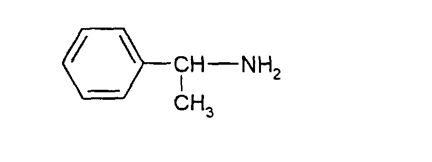

Die racemischen 1-Phenyl-ethylamine der Formel (I) sind bekannt (vgl. EP-A 0 341 475).

Die bei der Durchführung des erfindungsgemäßen Verfahrens als Reaktionskomponente benötigte (S)-(-)-N-Phenylcarbamat-milchsäure der Formel (II) ist ebenfalls bekannt (vgl. EP-A 0 341 475).

Als Kohlenwasserstoffe kommen sowohl bei der Durchführung der ersten als auch der zweiten Stufe des erfindungsgemäßen Verfahrens vorzugsweise Toluol oder Methylcyclohexan in Betracht. Als niederer aliphatischer Alkohol kommt sowohl für die Umsetzung in der ersten als auch in der zweiten Stufe vorzugsweise Methanol in Frage.

Als Alkalilaugen zur Freisetzung der (R)-1-Phenyl-ethylamine aus den in der ersten Stufe anfallenden Salzen kommen vorzugsweise wäßrige Natronlauge oder wäßrige Kalilauge in Betracht.

Die Reaktionstemperaturen können bei der Durchführung des erfindungsgemäßen Verfahrens innerhalb eines bestimmten Bereiches variiert werden. Sowohl bei der Durchführung der ersten als auch der zweiten Stufe arbeitet man im allgemeinen bei Temperaturen zwischen -20°C und +40°C, vorzugsweise zwischen 10°C und 40°C. Beim Entfernen des niederen aliphatischen Alkohols erwärmt man das Reaktionsgemisch im allgemeinen bis auf eine Temperatur von 40°C, vorzugsweise bis auf 30°C. Bei der Freisetzung des jeweiligen (R)-1-Phenyl-ethylamins aus dem entsprechenden Salz arbeitet man im allgemeinen bei Temperaturen zwischen 0°C und 50°C, vorzugsweise zwischen 0°C und 30°C.

Sowohl bei der Durchführung der ersten als auch der zweiten Stufe des erfindungsgemäßen Verfahrens arbeitet man im allgemeinen unter Atmosphärendruck. Beim Entfernen des niederen aliphatischen Alkohols arbeitet man jedoch vorzugsweise unter vermindertem Druck.

Bei der Durchführung des erfindungsgemäßen Verfahrens kann das Verhältnis der Reaktionskomponenten in einem bestimmten Bereich variiert werden. Auf 1 Mol an racemischem 1-Phenyl-ethylamin der Formel (I) setzt man bei der Durchführung der ersten Stufe im allgemeinen zwischen 0,25 und 0,50 Mol, vorzugsweise zwischen 0,40 und 0,475 Mol an (S)-(-)-N-Phenylcarbamat-milchsäure der Formel (II) ein. Die Menge an Kohlenwasserstoff kann bei der Durchführung der ersten Stufe des erfindungsgemäßen Verfahrens ebenfalls innerhalb eines bestimmten Bereiches variiert werden. Auf 1 Mol an racemischem 1-Phenyl-ethylamin der Formel (I) setzt man im allgemeinen zwischen 0,5 und 2 kg, vorzugsweise zwischen 0,75 und 1,5 kg an Kohlenwasserstoff ein.

Die Reaktionskomponenten können bei der Durchführung des erfindungsgemäßen Verfahrens in beliebiger Reihenfolge zusammengegeben werden. Die (S)-(-)-N-Phenylcarbamat-milchsäure der Formel (II) kann in fester Form oder auch in einem niederen aliphatischen Alkohol gelöst, gegebenenfalls im Gemisch mit einem aliphatischen oder aromatischen Kohlenwasserstoff, zugesetzt werden.

Der Vollständigkeit halber sei darauf hingewiesen, daß die (S)-(-)-N-Phenylcarbamat-milchsäure der Formel (II), die nach der Freisetzung des jeweiligen (R)-1-Phenyl-ethylamins in der Mutterlauge enthalten ist, durch Zugabe von verdünnter Mineralsäure, wie z.B. verdünnter Schwefelsäure oder verdünnter Salzsäure, ausgefällt und nach dem Abfiltrieren und Trocknen erneut eingesetzt werden kann. In entsprechender Weise läßt sich die (S)-(-)-N-Phenylcarbamatmilchsäure aus dem in der zweiten Stufe anfallenden Salz des racemischen 1-Phenyl-ethylamins isolieren.

Die nach dem erfindungsgemäßen Verfahren herstellbaren (R)-l-Phenyl-ethylamine der Formel (I-R) sind wertvolle Zwischenprodukte zur Synthese von Wirkstoffen mit fungiziden Eigenschaften (vgl. EP-A 0 341 475). So läßt sich das Diastereomerengemisch aus N-(R)-[1-(4-Chlor-phenyl)-ethyl]-(1R)-2,2-dichlor-1-ethyl-3t-methyl-1r-cyclopropan-carbonsäureamid und N-(R)-[1-(4-Chlor-phenyl)-ethyl]-(1S)-2,2-dichlor-1-ethyl-3t-methyl-1r-cyclopropan-carbonsäureamid der Formeln 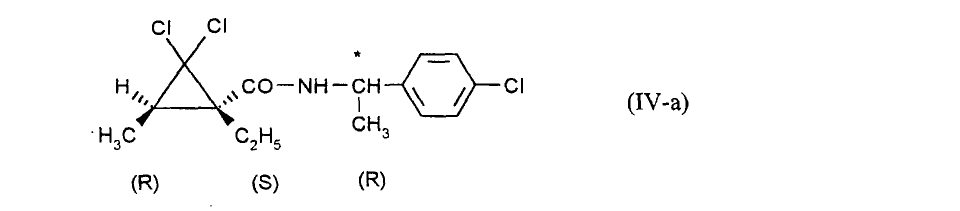 und 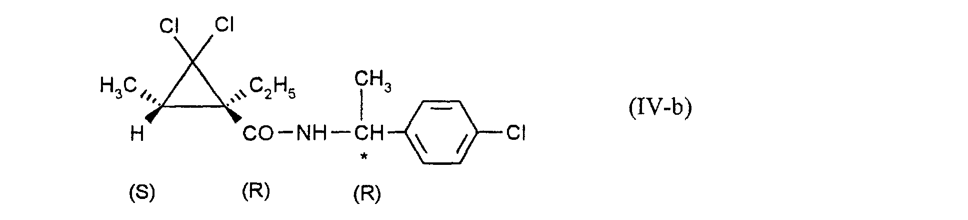 herstellen, indem man ein 1:1-Gemisch aus (1R)-2,2-Dichlor-1-ethyl-3t-methyl-1r-cyclopropan-carbonsäurechlorid und (1S)-2,2-Dichlor-1-ethyl-3t-methyl-1r-cyclopropan-carbonsäurechlorid der Formeln 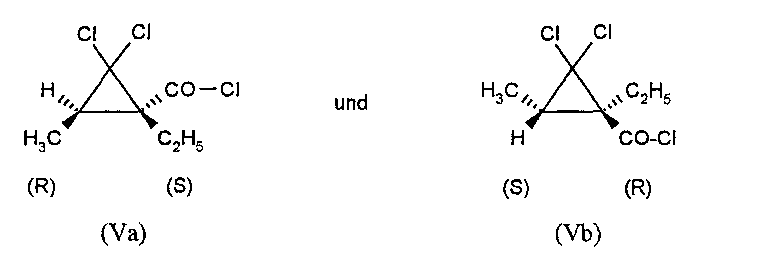 mit (R)-1-(4-Chlor-phenyl)-ethylamin der Formel 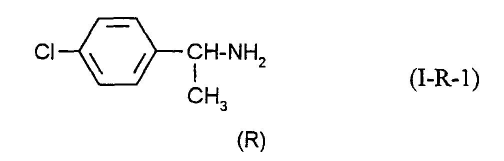 in Gegenwart eines Verdünnungsmittels, wie Methylenchlorid, und in Gegenwart eines Säurebindemittels, wie Triethylamin, umsetzt.

Die nach dem erfindungsgemäßen Verfahren weiterhin herstellbaren (S)-1-Phenylethylamine der Formel (I-S) sind ebenfalls wertvolle Zwischenprodukte. Sie lassen sich zur Synthese von Stoffen mit fungiziden oder pharmakologischen Eigenschaften einsetzen (vgl. EP-A 0 257 448 und EP-A 0 300 313). Außerdem können die (S)-1-Phenyl-ethylamine der Formel (I-S) auch racemisiert und erneut zur Racematspaltung eingesetzt werden (vgl. EP-A 0 489 682).

Die Durchführung des erfindungsgemäßen Verfahrens wird durch die folgenden Beispiele veranschaulicht.

### Herstellungsbeispiele

### Beispiel 1

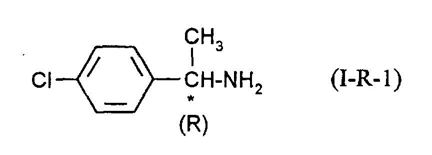

### Umsetzung (a)

In ein Gemisch aus 120 kg Methanol und 80 kg Toluol werden bei Raumtemperatur 18,8 kg (90 Mol) (S)-(-)-N-Phenylcarbamat-milchsäure gegeben. Das Gemisch wird unter Rühren auf 30°C erwärmt und mit 31,1 kg (200 Mol) an racemischem 1-(4-Chlorphenyl)-ethylamin versetzt. Danach destilliert man bei einer Sumpftemperatur von konstant 30°C unter allmählicher Reduzierung des Druckes auf 90 bis 100 mbar solange ein Gemisch aus Methanol und Toluol ab, bis die gaschromatographische Kontrolle ergibt, daß der Sumpf frei von Methanol ist. Während des Abdestillierens werden gleichzeitig 200 kg Toluol zugegeben, um die sich bildende Suspension gut rührbar und das Volumen des Reaktionsgemisches annähernd konstant zu halten. Anschließend wird das Reaktionsgemisch auf 0°C gekühlt und abgesaugt. Der anfallende Feststoff wird zweimal mit je 45 kg Toluol gewaschen und dann getrocknet. Man erhält auf diese Weise 32,8 kg an Produkt, das zu 95 % aus dem Salz des (R)-1-(4-Chlor-phenyl)-ethylamins mit der (S)-(-)-N-Phenylcarbamat-milchsäure besteht.

### Freisetzung von (R)-1-(4-Chlor-phenyl)-ethylamin

Das zuvor erwähnte Salz wird bei Raumtemperatur in Gegenwart von Toluol mit 95 kg 4 %iger wäßriger Natronlauge gerührt. Nach vollständiger Lösung des Salzes wird die organische Phase abgetrennt. Die wäßrige Phase wird zweimal mit Toluol extrahiert. Die vereinigten organischen Phasen werden unter vermindertem Druck destilliert. Man erhält auf diese Weise 13,7 kg an (R)-1-(4-Chlor-phenyl)-ethylamin (R:S-Verhältnis) = 96 : 4).

### Rückgewinnung von (S)-(-)-N-Phenylcarbamat-milchsäure

Die bei der Freisetzung von (R)-1-(4-Chlorphenyl)-ethylamin verbleibende wäßrige Phase wird mit 12 kg wäßriger Salzsäure (30 %ig) bei Raumtemperatur versetzt. Das dabei ausfallende Festprodukt wird abgesaugt und getrocknet. Man erhält auf diese Weise 18,2 kg an (S)-(-)-N-Phenylcarbamat-milchsäure.

### Aufarbeitung der Mutterlauge

Die bei der Umsetzung (a) anfallende Mutterlauge wird mit dem Toluol vereinigt, das zum Waschen des Salzes verwendet wurde. Aus dem Gemisch wird Toluol unter vermindertem Druck abdestilliert.

### Beispiel 2

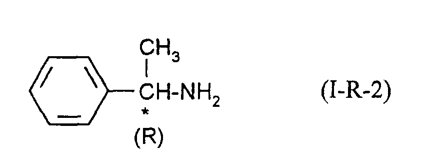

In ein Gemisch aus 230 g Toluol und 120 g Methanol werden bei Raumtemperatur unter Rühren 24,2 g (0,2 Mol) racemisches 1-Phenyl-ethylamin und 17,8 g (0,085 Mol) (S)-(-)-N-Phenylcarbamat-milchsäure gegeben. Das entstehende Gemisch wird 1 Stunde bei 30°C gerührt. Danach destilliert man bei einer Sumpftemperatur von maximal 30°C unter allmählicher Reduzierung des Druckes 230 g Lösungsmittel ab. Anschließend wird das Reaktionsgemisch auf Raumtemperatur abgekühlt und abgesaugt. Der anfallende Feststoff wird mit wenig Toluol gewaschen und getrocknet. Man erhält auf diese Weise 31 g an Produkt, das gemäß gaschromatographischer Analyse zu 81 % aus dem (R)-1-Phenyl-ethylamin-Salz der (S)-(-)-N-Phenylcarbamat-milchsäure und zu 19 % aus dem (S)-1-Phenylethylamin-Salz der (S)-(-)-N-Phenylcarbamat-milchsäure besteht.

Die nach der Abtrennung des Feststoffes anfallende, toluolhaltige Mutterlauge ist frei von (S)-(-)-N-Phenylcarbamat-milchsäure und enthält die restlichen Anteile des 1-Phenyl-ethylamins mit einem S:R-Isomerenverhältnis von 77,5:22,5.

### Beispiel 3

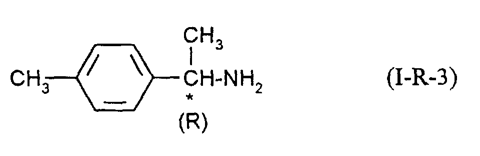

In ein Gemisch aus 230 g Toluol und 120 g Methanol werden bei 30°C unter Rühren 17,8 g (0,085 Mol) (S)-(-)-N-Phenylcarbamat-milchsäure und 27,0 g (0,2 Mol) racemisches 1-(4-Methyl-phenyl)-ethylamin gegeben. Nach einstündigem Nachrühren des Gemisches bei 30°C destilliert man bei einer Sumpftemperatur von maximal 30°C unter allmählicher Reduzierung des Druckes 229,7 g Lösungsmittel ab. Anschließend wird das Reaktionsgemisch auf Raumtemperatur abgekühlt und abgesaugt. Der anfallende Feststoff wird mit Toluol gewaschen und getrocknet. Man erhält auf diese Weise in quantitativer Ausbeute, bezogen auf eingesetzte (S)-(-)-N-Phenylcarbamat-milchsäure, das (R)-1-(4-Methyl-phenyl)-ethylamin-Salz der (S)-(-)-N-Phenylcarbamat-milchsäure, das gemäß gaschromatographischer Analyse eine optische Reinheit von 100 % besitzt.

Die nach der Abtrennung des Feststoffes anfallende Mutterlauge enthält das nicht als Salz gefällte (S)-1-(4-Methyl-phenyl)-ethylamin mit einem S:R-Isomerenverhältnis von 90,1:9,9.

### Beispiel 4

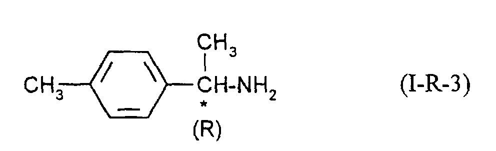

In ein Gemisch aus 120 g Methanol und 80 g Toluol werden bei Raumtemperatur unter Rühren 18,8 g (0,09 Mol) (S)-(-)-N-Phenylcarbamat-milchsäure gegeben. Das entstehende Gemisch wird bei 30°C unter Rühren mit 24,2 g (0,2 Mol) an racemischem 1-(4-Methyl-phenyl)-ethylamin versetzt. Danach destilliert man bei einer Sumpftemperatur von konstant 30°C unter allmählicher Reduzierung des Druckes so lange ein Gemisch aus Methanol und Toluol ab, bis der Sumpf frei von Methanol ist. Während des Abdestillierens werden gleichzeitig 200 g Toluol zugegeben, um die sich bildende Suspension gut rührbar zu halten. Anschließend wird das Reaktionsgemisch auf 10°C gekühlt und abgesaugt. Der anfallende Feststoff wird mit Toluol gewaschen und getrocknet. Man erhält auf diese Weise in quantitativer Ausbeute, bezogen auf die eingesetzte (S)-(-)-N-Phenylcarbamatmilchsäure ein Salz, das zu 97,2 % aus dem (R)-1-(4-Methyl-phenyl)-ethylamin-Salz der (S)-(-)-N-Phenylcarbamat-milchsäure und zu 2,8 % aus dem (S)-1-(4-Methyl-phenyl)-ethylamin-Salz der (S)-(-)-N-Phenylcarbamat-milchsäure besteht.

Die nach der Abtrennung des Feststoffes anfallende Mutterlauge enthält das nicht als Salz gefällte (S)-1-(4-Methyl-phenyl)-ethylamin mit einem S:R-Isomerenverhältnis von 89,1:10,9.

### Beispiel 5

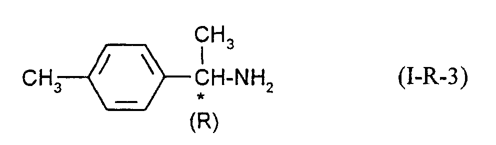

Nach der im Beispiel 4 angegebenen Methode werden 19,9 g (0,095 Mol) (S)-(-)-N-Phenylcarbamat-milchsäure mit 24,2 g (0,2 Mol) an racemischem 1-(4-Methyl-phenyl)-ethylamin umgesetzt. Man erhält in quantitativer Ausbeute, bezogen auf die eingesetzte (S)-(-)-N-Phenylcarbamat-milchsäure, ein Salz, das zu 94,2 % aus dem (R)-1-(4-Methyl-phenyl)-ethylamin-Salz der (S)-(-)-N-Phenylcarbamat-milchsäure und zu 5,8 % aus dem (S)-1-(4-Methyl-phenyl)-ethylamin-Salz der (S)-(-)-N-Phenylcarbamat-milchsäure besteht.

Die nach der Abtrennung des Feststoffes anfallende Mutterlauge enthält das nicht als Salz gefällte (S)-1-(4-Methyl-phenyl)-ethylamin mit einem S:R-Isomerenverhältnis von 90,7:9,3.

### Beispiel 6

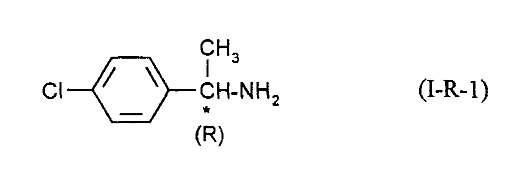

Nach der im Beispiel 1 angegebenen Methode werden jeweils 0,2 Mol racemisches 1-(4-Chlor-phenyl)-ethylamin mit
- 0,065 Mol,
- 0,07 Mol,
- 0,075 Mol,
- 0,08 Mol,
- 0,085 Mol bzw.
- 0,09 Mol an (S)-(-)-N-Phenylcarbamat-milchsäure umgesetzt. Nach der Aufarbeitung erhält man jeweils in quantitativer Ausbeute, bezogen auf die eingesetzte (S)-(-)-N-Phenylcarbamat-milchsäure, das (R)-1-(4-Chlor-phenyl)-ethylamin in einer optischen Reinheit von 96 bis 97 %.

Die nach der Abtrennung des Feststoffes anfallenden Mutterlaugen enthalten das nicht als Salz gefällte (S)-1-(4-Chlor-phenyl)-ethylamin in optischen Reinheiten zwischen 81 und 90 %.

### Beispiel 7

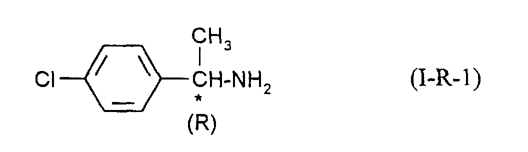

Unter Rühren bei Raumtemperatur werden 20,7 g (0,133 Mol) racemisches 1-(4-Chlor-phenyl)-ethylamin und 24,3 g (0,067 Mol) 1-(4-Chlor-phenyl)-ethylamin-Salz der (S)-(-)-N-Phenylcarbamat-milchsäure, das 0,028 Mol des (R)-Isomeren und 0,039 Mol des (S)-Isomeren des Amins enthält, in 413,9 g Methylcyclohexan gegeben. Durch Zugabe von 130 g Methanol und Erwärmen auf 40°C bildet sich eine klare, zweiphasige Lösung, die 0,2 Mol 1-(4-Chlor-phenyl)-ethylamin (0,095 Mol (R)-Isomeres und 0,105 Mol (S)-Isomeres) und 0,067 Mol (S)-(-)-N-Phenylcarbamat-milchsäure enthält. Danach destilliert man bei einer Sumpftemperatur von 40°C unter allmählicher Reduzierung des Druckes so lange ein Gemisch aus Methanol und Methylcyclohexan ab, bis der Sumpf frei von Methanol ist. Anschließend wird das Reaktionsgemisch auf Raumtemperatur abgekühlt und abgesaugt. Der anfallende Feststoff wird mit Toluol gewaschen und getrocknet. Man erhält auf diese Weise in quantitativer Ausbeute, bezogen auf die eingesetzte (S)-(-)-N-Phenylcarbamat-milchsäure, das (R)-1-(4-Chlor-phenyl)-ethylamin-Salz der (S)-(-)-N-Phenylcarbamat-milchsäure in einer optischen Reinheit von 98,1 %.

### Beispiel 8

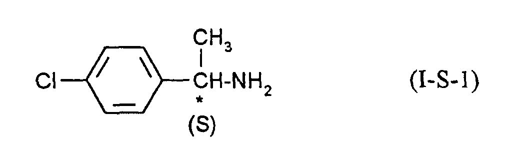

Nach der im Beispiel 1 angegebenen Methode werden 31,1 g (0,2 Mol) racemisches 1-(4-Chlor-phenyl)-ethylamin mit 18,8 g (0,09 Mol) (S)-(-)-N-Phenylcarbamat-milchsäure in Gegenwart von 413,9 g Toluol und 117,5 g Methanol umgesetzt. Danach destilliert man bei einer Sumpftemperatur von maximal 30°C unter allmählicher Reduzierung des Druckes so lange ein Gemisch aus Methanol und Toluol ab, bis der Sumpf frei von Methanol ist. Während des Abdestillierens wird gleichzeitig soviel Toluol zugefügt, daß die entstehende Suspension gut rührbar bleibt. Man kühlt das Reaktionsgemisch auf 0°C und saugt den anfallenden Feststoff ab.

Die Mutterlauge wird bei 40°C unter Rühren mit 4,2 g (0,02 Mol) (S)-(-)-N-Phenylcarbamat-milchsäure und 19 g Methanol versetzt. Danach destilliert man bei einer Sumpftemperatur von maximal 40°C unter allmählicher Reduzierung des Druckes so lange ein Gemisch aus Methanol und Toluol ab, bis der Sumpf frei von Methanol ist. Aus der entstehenden Suspension wird der Feststoff abgesaugt und getrocknet. Man erhält auf diese Weise 7,3 g an Salz aus racemischem 1-(4-Chlor-phenyl)-ethylamin und (S)-(-)-N-Phenylcarbamat-milchsäure. Durch destillative Aufarbeitung der Mutterlauge erhält man 15,5 g (0,1 Mol) (S)-1-(4-Chlor-phenyl)-ethylamin in einer optischen Reinheit von 96,1 %.

### Beispiel 9

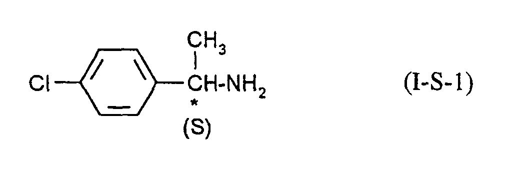

Nach der im Beispiel 8 angegebenen Methode werden jeweils 31,1 g (0,2 Mol) racemisches 1-(4-Chlor-phenyl)-ethylamin mit
- 0,065 Mol,
- 0,07 Mol,
- 0,075 Mol,
- 0,08 Mol bzw.
- 0,085 Mol an (S)-(-)-N-Phenylcarbamat-milchsäure umgesetzt. Die nach der Abtrennung des Kristallisats verbleibende Mutterlauge wird mit jeweils einer solchen Menge an (S)-(-)-N-Phenylcarbamat-milchsäure versetzt, die dem Doppelten der Differenz aus 0,1 Mol und dem anfänglichen molaren Einsatz an (S)-(-)-N-Phenylcarbamat-milchsäure entspricht. Dabei wird die (S)-(-)-N-Phenylcarbamat-milchsäure vor der Zugabe jeweils in Methanol gelöst und zwar werden auf 2,0 g (0,01 Mol) an (S)-(-)-N-Phenylcarbamat-milchsäure jeweils 10 g an Methanol verwendet.

Aus dem jeweiligen Reaktionsgemisch wird bei einer Sumpftemperatur von maximal 40°C unter allmählicher Reduzierung des Druckes so lange ein Gemisch aus Methanol und Toluol abdestilliert, bis der Sumpf frei von Methanol ist. Danach wird der entstandene Feststoff abgesaugt und getrocknet. Durch destillative Aufarbeitung der Mutterlaugen erhält man (S)-1-(4-Chlor-phenyl)-ethylamin in einer optischen Reinheit von
- 95,0 %,
- 96,0 %,
- 96,2 %,
- 97,9 % bzw.
- 96,4 %.

### Vergleichsbeispiel A

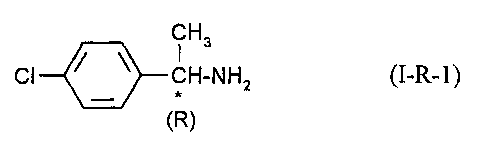

Nach der im Beispiel 1 angegebenen Methode werden jeweils 31,1 g (0,2 Mol) racemisches 1-(4-Chlor-phenyl)-ethylamin mit
- 0,095 Mol,
- 0,1 Mol,
- 0,105 Mol bzw.
- 0,11 Mol an (S)-(-)-N-Phenylcarbamat-milchsäure umgesetzt. Nach der Aufarbeitung in der zuvor geschilderten Weise erhält man jeweils in quantitativer Ausbeute, bezogen auf die eingesetzte (S)-(-)-N-Phenylcarbamat-milchsäure, das (R)-1-(4-Chlor-phenyl)-ethylamin-Salz der (S)-(-)-N-Phenylcarbamat-milchsäure. Die optischen Reinheiten des daraus freigesetzten (R)-1-(4-Chlor-phenyl)-ethylamins liegen zwischen 70 und 80 %.

## Patentansprüche

1. Verfahren zur Herstellung von optisch aktiven 1-Phenyl-ethylaminen der Formel 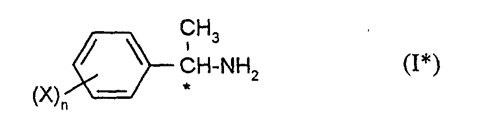 in welcher
X für Wasserstoff, Halogen oder Methyl steht und
n für die Zahlen 0, 1 oder 2 steht,
aus racemischen 1-Phenyl-ethyl-aminen mit Hilfe von (S)-(-)-N-Phenylcarbamat-milchsäure,
**dadurch gekennzeichnet, daß** man
a) racemische 1-Phenyl-ethylamine der Formel 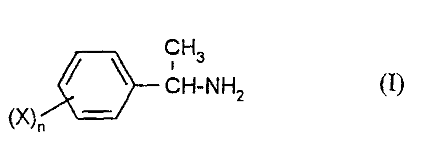 in welcher
X und n die oben angegebenen Bedeutungen haben,
mit (S)-(-)-N-Phenylcarbamat-milchsäure der Formel 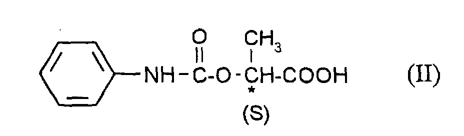 in Gegenwart eines aliphatischen oder aromatischen Kohlenwasserstoffes und in Gegenwart eines niederen aliphatischen Alkohols umsetzt, wobei die Mengen an den Reaktionskomponenten so bemessen sind, daß auf 1 Mol an racemischem 1-Phenyl-ethyl-amin der Formel (I) zwischen 0,25 und 0,5 Mol an (S)-(-)-N-Phenylcarbamat-milchsäure der Formel (II) vorhanden sind,
danach den niederen aliphatischen Alkohol sowie gegebenenfalls einen Teil des aliphatischen oder aromatischen Kohlenwasserstoffes bei einer Sumpftemperatur bis zu 40°C gegebenenfalls unter vermindertem Druck abdestilliert,
das dabei entstehende kristalline Produkt der Formel 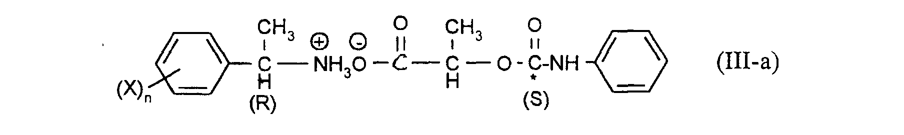 in welcher
X und n die oben angegebenen Bedeutungen haben,
abtrennt, mit verdünnter, wäßriger Alkalilauge in Gegenwart eines aliphatischen oder aromatischen Kohlenwasserstoffes verrührt, die organische Phase abtrennt, die wäßrige Phase erneut mit einem aliphatischen oder aromatischen Kohlenwasserstoff verrührt, die organische Phase wiederum abtrennt und aus den vereinigten organischen Phasen das jeweils entstandene (R)-1-Phenyl-ethylamin der Formel 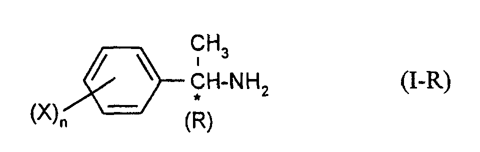 in welcher
X und n die oben angegebenen Bedeutungen haben,
destillativ isoliert,
und gegebenenfalls
b) die nach der Abtrennung des Salzes der Formel (III-a) verbliebene Mutterlauge in Gegenwart eines niederen aliphatischen Alkohols mit (S)-(-)-N-Phenylcarbamat-milchsäure der Formel 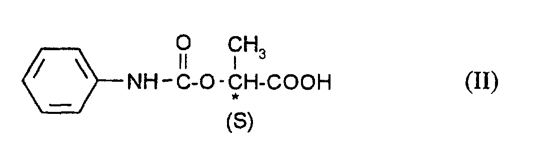 umsetzt, wobei die Mengen an den Reaktionskomponenten so bemessen sind, daß die molare Menge an (S)-(-)-N-Phenylcarbamatmilchsäure der Formel (II) doppelt so groß ist wie die in der Mutterlauge noch vorhandene Menge an (R)-1-Phenyl-ethylamin der Formel (I-R), danach den niederen aliphatischen Alkohol sowie gegebenenfalls einen Teil des aliphatischen oder aromatischen Kohlenwasserstoffes bei einer Sumpftemperatur bis zu 40°C gegebenenfalls unter vermindertem Druck abdestilliert,
das dabei entstehende kristalline Produkt der Formel 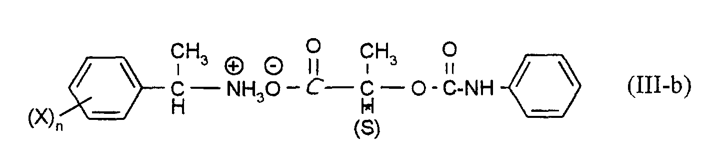 in welcher
X und n die oben angegebenen Bedeutungen haben,
abtrennt und aus der verbleibenden Mutterlauge das jeweils entstandene (S)-1-Phenyl-ethylamin der Formel 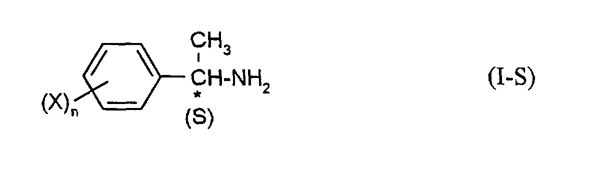 in welcher
X und n die oben angegebenen Bedeutungen haben,
destillativ isoliert.

2. Verfahren gemäß Anspruch 1, **dadurch gekennzeichnet, daß** man als Ausgangsstoffe racemische 1-Phenyl-ethylamine der Formel (I) einsetzt, in denen
X für Fluor, Chlor, Brom oder Methyl steht und
n für die Zahlen 0, 1 oder 2 steht.

3. Verfahren gemäß Anspruch 1, **dadurch gekennzeichnet, daß** man als Ausgangsstoff racemisches 1-(4-Chlor-phenyl)-ethylamin einsetzt.

4. Verfahren gemäß Anspruch 1, **dadurch gekennzeichnet, daß** man als Ausgangsstoff racemisches 1-(2,4-Dichlor-phenyl)-ethylamin einsetzt.

5. Verfahren gemäß Anspruch 1, **dadurch gekennzeichnet, daß** man als Kohlenwasserstoffe Toluol oder Methylcyclohexan einsetzt.

6. Verfahren gemäß Anspruch 1, **dadurch gekennzeichnet, daß** man als niederen aliphatischen Alkohol Methanol einsetzt.

7. Verfahren gemäß Anspruch 1, **dadurch gekennzeichnet, daß** man als Alkalilauge wäßrige Natronlauge oder wäßrige Kalilauge einsetzt.

8. Verfahren gemäß Anspruch 1, **dadurch gekennzeichnet, daß** man sowohl bei der Durchführung der ersten als auch der zweiten Stufe bei Temperaturen zwischen -20°C und +40°C arbeitet.

9. Verfahren gemäß Anspruch 1, **dadurch gekennzeichnet, daß** man beim Einengen des Reaktionsgemisches unter vermindertem Druck und bei einer Sumpftemperatur von maximal 30°C arbeitet.

10. Verfahren gemäß Anspruch 1, **dadurch gekennzeichnet, daß** man bei der Durchführung der ersten Stufe die Mengen an den Reaktionskomponenten so bemißt, daß man auf 1 Mol an racemischem 1-Phenyl-ethylamin der Formel (I) zwischen 0,40 und 0,475 Mol an (S)-(-)-N-Phenylcarbamatmilchsäure der Formel (II) einsetzt.

## Claims

1. Process for the preparation of optically active 1-phenyl-ethylamines of the formula 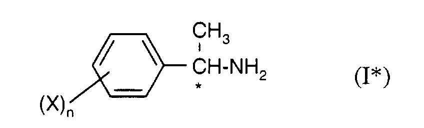 in which
X is hydrogen, halogen or methyl, and
n is 0, 1 or 2,
from racemic 1-phenyl-ethyl-amines using (S)-(-)-N-phenylcarbamate lactic acid,
**characterized in that**
a) racemic 1-phenyl-ethylamines of the formula 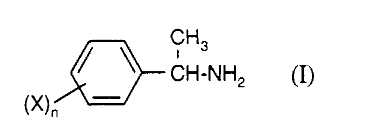 in which
X and n are as defined above
are reacted with (S)-(-)-N-phenylcarbamate lactic acid of the formula 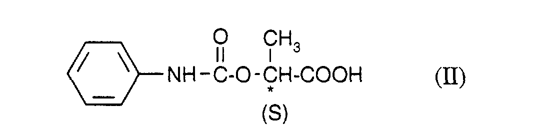 in the presence of an aliphatic or aromatic hydrocarbon and in the presence of a lower aliphatic alcohol, the amounts of the reaction components being such that per 1 mol of racemic 1-phenyl-ethyl-amine of the formula (I), between 0.25 and 0.5 mol of (S)-(-)-N-phenylcarbamate lactic acid of the formula (II) are present,
then the lower aliphatic alcohol and, where appropriate, some of the aliphatic or aromatic hydrocarbon are distilled off at a still temperature up to 40°C, optionally under reduced pressure,
the resulting crystalline product of the formula 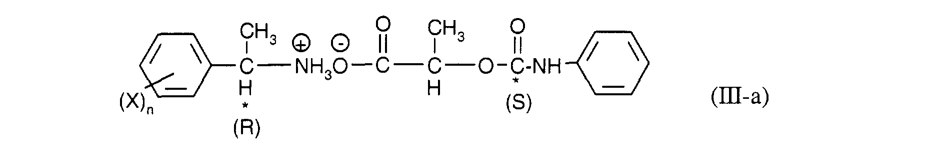 in which
X and n are as defined above,
is separated off, stirred with dilute, aqueous alkaline solution in the presence of an aliphatic or aromatic hydrocarbon, the organic phase is separated off, the aqueous phase is again stirred with an aliphatic or aromatic hydrocarbon, the organic phase is again separated off and the combined organic phases are distilled to isolate the (R)-1-phenylethylamine of the formula 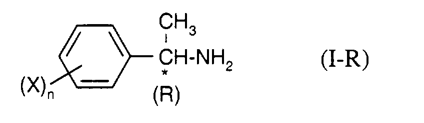 in which
X and n are as defined above
which is produced in each case
and optionally
b) the mother liquor which remains after the salt of the formula (III-a) has been separated off is reacted, in the presence of a lower aliphatic alcohol, with (S)-(-)-N-phenylcarbamate lactic acid of the formula 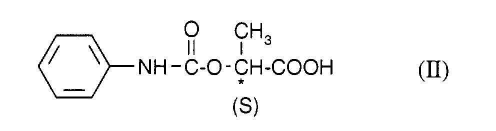 where the amounts of the reaction components are such that the molar amount of (S)-(-)-N-phenylcarbamate lactic acid of the formula (II) is twice the amount of (R)-1-phenyl-ethylamine of the formula (I-R) still present in the mother liquor, then the lower aliphatic alcohol and, where appropriate, some of the aliphatic or aromatic hydrocarbon, are distilled at a still temperature up to 40°C, optionally under reduced pressure,
the resulting crystalline product of the formula 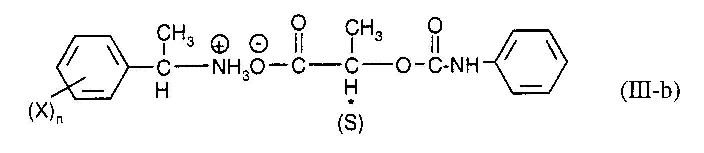 in which
X and n are as defined above
is separated off, and the mother liquor which remains is distilled to isolate the (S)-1-phenyl-ethylamine of the formula 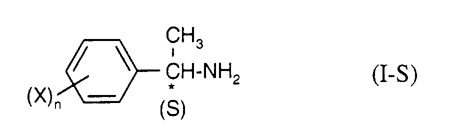 in which
X and n are as defined above,
which is produced in each case.

2. Process according to Claim 1, **characterized in that** the starting materials used are racemic 1-phenyl-ethylamines of the formula (I) in which
X is fluorine, chlorine, bromine or methyl, and
n is 0, 1 or 2.

3. Process according to Claim 1, **characterized in that** the starting material used is racemic 1-(4-chloro-phenyl)-ethylamine.

4. Process according to Claim 1, **characterized in that** the starting material used is racemic 1-(2,4-dichloro-phenyl)-ethylamine.

5. Process according to Claim 1, **characterized in that** the hydrocarbons used are toluene or methylcyclohexane.

6. Process according to Claim 1, **characterized in that** the lower aliphatic alcohol used is methanol.

7. Process according to Claim 1, **characterized in that** the alkaline solution used is aqueous sodium hydroxide solution or aqueous potassium hydroxide solution.

8. Process according to Claim 1, **characterized in that** both the first and also the second stage is carried out at temperatures between -20°C and +40°C.

9. Process according to Claim 1, **characterized in that** the reaction mixture is evaporated under reduced pressure and at a still temperature of at most 30°C.

10. Process according to Claim 1, **characterized in that** the first stage is carried out using amounts of the reaction components such that per 1 mol of racemic 1-phenyl-ethylamine of the formula (I), between 0.40 and 0.475 mol of (S)-(-)-N-phenylcarbamate lactic acid of the formula (II) is used.

## Revendications

1. Procédé de production de 1-phényléthylamines optiquement actives de formule 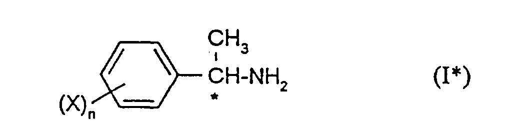 dans laquelle
X représente l'hydrogène, un halogène ou le groupe méthyle et
n représente les nombres 0, 1 ou 2,
à partir de 1-phényléthylamines racémiques à l'aide d'acide (S)-(-)-N-phénylcarbamate-lactique,
**caractérisé en ce que**
a) on fait réagir des 1-phényléthylamines racémiques de formule 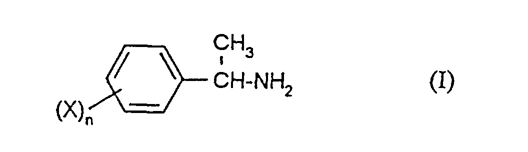 dans laquelle
X et n ont les définitions indiquées ci-dessus,
avec l'acide (S)-(-)-N-phénylcarbamate-lactique de formule 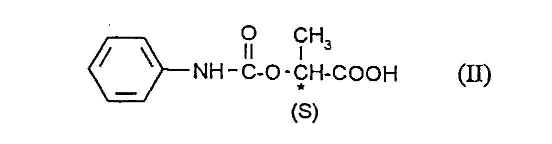 en présence d'un hydrocarbure aliphatique ou aromatique et en présence d'un alcool aliphatique inférieur, en calculant les quantités de composants réactionnels de manière qu'il y ait, par mole de 1-phényléthylamine racémique de formule (I), entre 0,25 et 0,5 mole d'acide (S)-(-)-N-phénylcarbamate-lactique de formule (II),
puis on chasse par distillation l'alcool aliphatique inférieur ainsi que, le cas échéant, une partie de l'hydrocarbure aliphatique ou aromatique à une température au bas de la colonne allant jusqu'à 40°C, éventuellement sous pression réduite,
on sépare le produit cristallin de formule 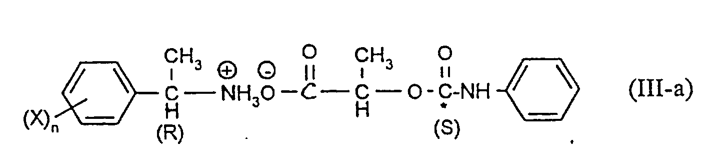 dans laquelle
X et n ont les définitions indiquées ci-dessus,
qui est alors formé, on le délaye avec une lessive alcaline aqueuse diluée en présence d'un hydrocarbure aliphatique ou aromatique, on sépare la phase organique, on délaye de nouveau la phase aqueuse avec un hydrocarbure aliphatique ou aromatique, on sépare de nouveau la phase organique et on isole par distillation des phases organiques rassemblées la (R)-1-phényléthylamine produite dans chaque cas, de formule 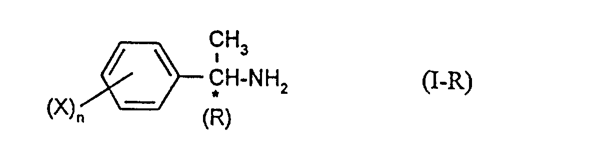 dans laquelle
X et n ont les définitions indiquées ci-dessus,
et, le cas échéant,
b) on fait réagir la liqueur-mère restant après séparation du sel de formule (III-a) en présence d'un alcool aliphatique inférieur avec l'acide (S)-(-)-N-phénylcarbamate-lactique de formule 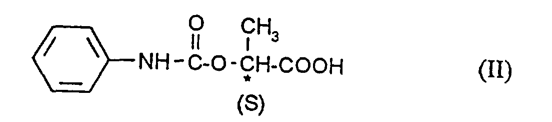 en calculant les quantités de composants réactionnels de manière que la quantité molaire d'acide (S)-(-)-N-phénylcarbamate-lactique de formule (II) soit double de la quantité de (R)-1-phényléthylamine de formule (I-R) encore présente dans la liqueur-mère, puis on chasse par distillation éventuellement sous pression réduite l'alcool aliphatique inférieur ainsi que, le cas échéant, une partie de l'hydrocarbure aliphatique ou aromatique à une température au bas de la colonne allant jusqu'à 40°C,
on sépare le produit cristallin de formule 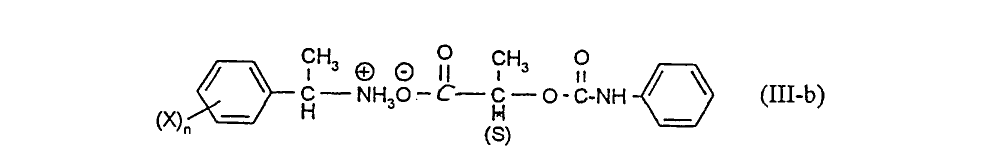 dans laquelle
X et n ont les définitions indiquées ci-dessus,
qui est alors formé et on isole de la liqueur-mère restante, par distillation, la (S)-1-phényléthylamine produite dans chaque cas, de formule 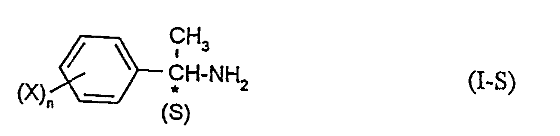 dans laquelle
X et n ont les définitions indiquées ci-dessus.

2. Procédé suivant la revendication 1, **caractérisé en ce qu'**on utilise comme composés de départ des 1-phényléthylamines racémiques de formule (I), dans lesquelles
X représente le fluor, le chlore, le brome ou le groupe méthyle et
n représente les nombres 0, 1 ou 2.

3. Procédé suivant la revendication 1, **caractérisé en ce qu'**on utilise comme composé de départ la 1-(4-chlorophényl)éthylamine racémique.

4. Procédé suivant la revendication 1, **caractérisé en ce qu'**on utilise comme composé de départ la 1-(2,4-dichlorophényl)éthylamine racémique.

5. Procédé suivant la revendication 1, **caractérisé en ce qu'**on utilise comme hydrocarbures le toluène ou le méthylcyclohexane.

6. Procédé suivant la revendication 1, **caractérisé en ce qu'**on utilise le méthanol comme alcool aliphatique inférieur.

7. Procédé suivant la revendication 1, **caractérisé en ce qu'**on utilise comme lessive alcaline la lessive de soude aqueuse ou la lessive de potasse aqueuse.

8. Procédé suivant la revendication 1, **caractérisé en ce qu'**on opère à des températures comprises entre -20°C et +40°C dans la conduite tant de la première que de la seconde étape.

9. Procédé suivant la revendication 1, **caractérisé en ce qu'**on opère sous pression réduite et à une température au bas de la colonne au maximum égale à 30°C lors de la concentration du mélange réactionnel.

10. Procédé suivant la revendication 1, **caractérisé en ce que** dans la conduite de la première étape, on calcule les quantités de composants réactionnels de manière à utiliser, par mole de 1-phényléthylamine racémique de formule (I), entre 0,40 et 0,475 mole d'acide (S)-(-)-N-phénylcarbamate-lactique de formule (II).
